# EUROPEAN PATENT SPECIFICATION

(11) **EP 3 375 439 B1**
(45) Date of publication and mention of the grant of the patent: **07.10.2020**
(21) Application number: 16864473.0
(22) Date of filing: 20.10.2016
(51) Int. Cl.: A61K 31/047, A61K 31/075, A23L 33/10, A61P 5/24

(54) **COMPOSITION FOR ALLEVIATING, PREVENTING OR TREATING PREMENSTRUAL SYNDROME, CONTAINING PINITOL OR D-CHIRO-INOSITOL**
ZUSAMMENSETZUNG ZUR LINDERUNG, PRÄVENTION ODER BEHANDLUNG DES PRÄMENSTRUELLEN SYNDROMS ENTHALTEND PINITOL ODER D-CHIRO-INOSITOL
COMPOSITION VISANT À SOULAGER, PRÉVENIR OU TRAITER LE SYNDROME PRÉMENSTRUEL ET CONTENANT DU PINITOL, OU D-CHIRO-INOSITOL

(30) Priority: 09.11.2015 KR 20150156930
(43) Date of publication of application: 19.09.2018
(73) Proprietor: DY Natural Co., Ltd., Daejeon 34145 (KR)
(72) Inventor: MYEONG, Hyun Koon, Daejeon 34140 (KR); KIM, Kwang Kyu, Daejeon 35251 (KR)
(74) Representative: Plougmann Vingtoft a/s
(86) International application number: PCT/KR2016/011806
(87) International publication number: WO 2017/082547

(56) References cited:
- EP-A1- 2 875 809
- GIANFRANCO CARLOMAGNO ET AL: "Myo-inositol in the treatment of premenstrual dysphoric disorder : MYO-INOSITOL IN THE TREATMENT OF PMDD", HUMAN PSYCHOPHARMACOLOGY. CLINICAL AND EXPERIMENTAL., vol. 26, no. 7, 1 October 2011 (2011-10-01), pages 526-530, XP055383511, XX ISSN: 0885-6222, DOI: 10.1002/hup.1241
- TOMOHIKO MUKAI ET AL: "A meta-analysis of inositol for depression and anxiety disorders : INOSITOL AND DEPRESSION AND ANXIETY DISORDERS", HUMAN PSYCHOPHARMACOLOGY. CLINICAL AND EXPERIMENTAL., vol. 29, no. 1, 3 December 2013 (2013-12-03), pages 55-63, XP055506609, XX ISSN: 0885-6222, DOI: 10.1002/hup.2369
- CARLOMAGNO, G. ET AL.: 'Myo-inositol in the Treatment of Premenstrual Dysphoric Disorder' HUMAN PSYCHOPHARMACOLOGY: CLINICAL AND EXPERIMENTAL vol. 26, 2011, pages 526 - 530, XP055383511
- NESTLER, J. E. ET AL.: 'Ovulatory and Metabolic Effects of D-chiro-inositol in the Polycystic Ovary Syndrome' THE NEW ENGLAND JOURNAL OF MEDICINE vol. 340, no. 17, 1999, pages 1314 - 1320, XP007906382
- GABY, A. R.: 'Case Report: D-pinitol for Polycystic Ovary Syndrome' TOWNSEND LETTER 2009, page 44, XP009506216
- NORDIO, M. ET AL.: 'The Combined Therapy with Myo-inositol and D-chiro-inositol Reduces the Risk of Metabolic Disease in PCOS Overweight Patients Compared to Myo-inositol Supplementation Alone' EUROPEAN REVIEW FOR MEDICAL AND PHARMACOLOGICAL SCIENCES vol. 16, 2012, pages 575 - 581, XP009168775
- RENGARAJAN, T. ET AL.: 'D-pinitol Promotes Apoptosis in MCF-7 Cells via Induction of p53 and Bax and Inhibition of Bcl-2 and NF-kappa B' ASIAN PACIFIC JOURNAL OF CANCER PREVENTION vol. 15, no. 4, 2014, pages 1757 - 1762, XP055383526

## Description

### Technical Field

The present patent application claims the benefit of priority to the Korean Patent Application No. 10-2015-0156930 filed in the Korean Intellectual Property Office on November 09, 2015. The present disclosure relates to a composition comprising pinitol or d-chiro-inositol as an effective ingredient for alleviating, preventing, or treating premenstrual syndrome

### Background Art

Generally, women undergo menstruation for about 35 years after starting the first menstrual period. When fertility in a female is inclined, menstruation occurs less regularly for months to years, followed by the final menstrual period between the ages of 45 to 55 (Woods NF , Mos s A and Dery GK (1982). Prevalence of perimenstrual symptoms. AJPH, 72(11), 1257-1264). Premenstrual syndrome refers to mental, behavioral, and physical symptoms that occur 7-10 days before onset of menstruation and completely disappear or reduce with onset of a women's period (Greene R, Dalton K (1953): The premenstrual syndrome. BMJ 1(4818): 1007-1014). Rather than premenstrual syndrome, pre- and postmenstrual syndrome may be a more accurate term because in addition to the development of physical and emotional changes near menstruation, various symptoms do not completely vanish, but persist for some women during and even after their periods.

About 150-200 different symptoms have been associated with premenstrual symptom. Representative among them are nervousness, mood swings, depression, tension, anxiety, fatigue, pain, low back pain, weight gain, mastalgia, and edema. Rare symptoms are reported for some women (Glas s GS, et al., 1971. Psychiatric emergency related of Psychiatry, 128, 705-711; Par lee MB, 1976. Primary Care, 3, 477- 489; Andersh B and Hahn L. Premenstrual complaints, Influence of oral contraceptives, Acta obst et gynec. scand., 60, 579-583; Sanders D, et al., 1983. Psychosomatic Medicine, 45 (6), 487- 516; Muse KN. Cet el NS, Futterman LA and Yen SCC(1984). New. Engl. J. Med., 311, 1345- 1369; Shaver JF and Woods NF (1985) . Research in Nursing and health, 8, 313, 319; Sigel J (1985). Health and Social Work , 12, 284- 289).

Premenstrual syndrome is suggested to have various causes including hormonal imbalance (Thys-Jacobs S, Alvir MJ (1995), J Clin Endocrinol Metab 80(7): 2227-2232), dietary factors such as malnutrition (Lauerson NH (1985): Nurse Pract 10(3): 11-17; Tayler RJ, Fordyce ID, Aleander DA (1991): Relationship between personality and premenstrual symptoms. Br J Gen Practice 41(3): 55-57), imbalanced neurotransmitters (O'Brien PM (1993): Helping women with premenstrual syndrome. BMJ 307(6917): 1471-1475), etc., and is accounted for by multifactorial neuropsychology-endocrine dysfunction. About 70-90 % of the women of child bearing ages report symptoms associated with menstruation, and about 20-50 % of all women have experienced premenstrual syndrome, with 2.5 % thereof suffering from severe symptoms enough to interfere with the woman's everyday life (Mortola J (1998): N Engl J Med 338(4): 256-257; Singh B, et al., (1998): Altern Ther Health Med 4(3): 75-79).

Pinitol is a compound isolated from Bougainvillea spectabilis, sugar pine (Pinus lambertiana), etc., and has a chemical structure and biochemical function similar to that of inositol. D-chiro-inositol (DCI) is a member of the inositol family. Pinitol is converted in vivo to D-chiro-inositol. Various prior art documents discloses that pinitol, D-chiro-inositol, and derivatives thereof have various biological activities including antidiabetes anti-inflammation, and anticancer effects (WO 96/29063, USP 5,827,896, USP 5,124,360).

Carlomagno et al. (Human Psychopharmacology: Clinical and Experimental 26.7 (2011): 526-530) relates to myo-inositol in the treatment of premenstrual dysphoric disorder.

### Detailed Description of the Invention

### Technical Problem

Leading to the present invention, intensive and thorough research, conducted by the present inventors, into naturally occurring active materials useful for alleviating and treating symptoms of premenstrual syndrome resulted in the finding that pinitol and D-chiro-inositol can remarkably reduce symptoms of premenstrual syndrome, such as dysmenorrhea, depression, etc. Accordingly, it is a purpose of the present disclosure to provide a pharmaceutical composition comprising pinitol or D-chiro-inositol as an effective ingredient for preventing or treating premenstrual syndrome.

It is another purpose of the present disclosure to provide a food composition comprising pinitol or D-chiro-inositol as an effective ingredient for use in preventing or alleviating premenstrual syndrome.

Further described herein is a method for preventing or treating premenstrual syndrome, the method comprising a step of injecting into a subject a pharmaceutical composition comprising pinitol, D-chiro-inositol, or an analog thereof as an effective ingredient.

Other objects and advantages of the present invention will become apparent from the following detailed description together with the appended claims and drawings

### Technical Solution

The present invention provides a pharmaceutical composition comprising pinitol or D-chiro-inositol as an effective ingredient for use in the prevention or treatment of premenstrual syndrome.

The invention also provides a food composition comprising pinitol or D-chiro-inositol as an effective ingredient for use in alleviating premenstrual syndrome.

The effective ingredient "pinitol" in the composition described herein is represented by the following Chemical Formula 1:

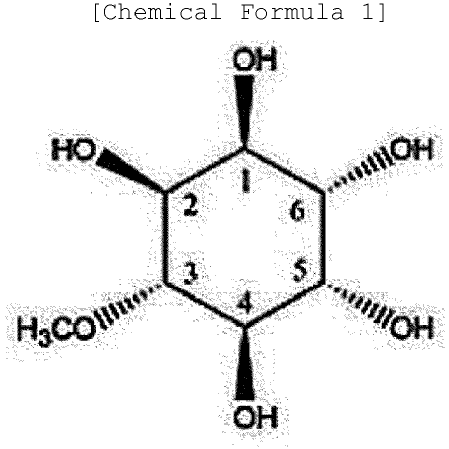

As used herein, the term "pinitol-like compound" or "analog of pinitol" is intended to encompass all compounds that have functional and structural features in common with pinitol and possess the biological activity equivalent to that of "pinitol" immediately or after in-vivo metabolism. Such pinitol-like compounds or analogs are not part of the present invention (neither are pinitol containing compounds as defined below, derivatives, metabolites, or pro-drugs of pinitol as defined below, or D-chiro-inositol-like compounds or analogs as defined below). The scope of the present invention is defined in the claims; any other disclosure in the present description, regardless of whether it is indicated as being preferred or exemplified, does not form part of the present invention.

In the present disclosure, the "pinitol-like compound" or "analog of pinitol" cover proper "derivatives" or "metabolites" of pinitol, "pinitol-containing compounds", or "prodrugs of pinitol".

Examples of the proper "derivatives" or "metabolites" of pinitol in the present disclosure include pinitol glycosides, pinitol phospholipids, esterified pinitol, lipid-bound pinitol, pinitol phosphate, pinitol phytates, or a mixture thereof, but are not limited thereto.

As used herein, the term "pinitol-containing compound" refers to a compound containing a pinitol moiety as a major portion thereof. The "pinitol-containing compound" includes polysaccharides containing pinitol and one or more additional sugars or sugar derivatives, or complexes or chelate compounds containing pinitol and one or more metal ions, but are not limited thereto.

The term "pro-drug of pinitol", as used herein, refers to a pinitol derivative that is converted in vivo to true pinitol by enzymatic or chemical processes and exhibits more improved transport features and/or therapeutic efficacy. Methods for preparation and administration of prodrugs of sugars, for example, methylated or acetylated sugars having hydroxyl groups are known in the art (Baker et al., J. Med. Chem., 27:270- 274, 1984).

Pinitol and pinitol-like compounds can be obtained through extraction and purification processes from pine leaves, chick peas, Bougainvillea leaves, alfalfa, carob, buckwheat, and Cucurbita ficifolia, or through synthesis processes.

The effective ingredient "D-chiro-inositol" in the composition described herein is represented by the following Chemical Formula 2.

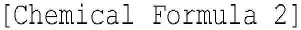

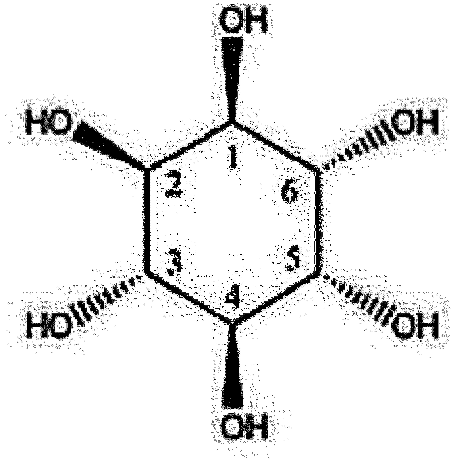

As used herein, the term "D-chiro-inositol-like compound" or "analog of D-chiro-inositol" is intended to encompass all compounds that have functional and structural features in common with D-chiro-inositol and possess the biological activity equivalent to that of "D-chiro-inositol" immediately or after in-vivo metabolism.

In the present disclosure, the "D-chiro-inositol-like compound" or "analog of D-chiro-inositol" cover proper "derivatives" or "metabolites" of D-chiro-inositol, "D-chiro-inositol-containing compounds", or "prodrugs of D-chiro-inositol".

Examples of the proper "derivatives" or "metabolites" of D-chiro-inositol in the present disclosure include D-chiro-inositol phosphate, D-chiro-inositol ester, D-chiro-inositol acetate, D-chiro-inositol ester, lower alkyl ethers of D-chiro-inositol, D-chiro-inositol acetal, and D-chiro-inositol ketal, but are not limited thereto.

As used herein, the term "D-chiro-inositol-containing compound" refers to a compound containing a D-chiro-inositol moiety as a major portion thereof.

The "D-chiro-inositol-containing compound" includes polysaccharides containing D-chiro-inositol and one or more additional sugars or sugar derivatives, D-chiro-inositol-phospholipid, or complexes or chelate compounds containing D-chiro-inositol and one or more metal ions, but is not limited thereto.

The term "pro-drug of D-chiro-inositol", as used herein, refers to a D-chiro-inositol derivative that is converted in vivo to true D-chiro-inositol by enzymatic or chemical processes and exhibits more improved transport features and/or therapeutic efficacy. Methods for preparation and administration of prodrugs of sugars, for example, methylated or acetylated sugars having hydroxyl groups are known in the art (Baker et al., J. Med. Chem., 27:270- 274, 1984).

D-chiro-inositol and D-chiro-inositol-like compounds can be obtained through extraction and purification processes from pine leaves, chick peas, Bougainvillea leaves, alfalfa, carob, buckwheat, and Cucurbita ficifolia, through treatment of myo-inositol with epimerase, or through synthesis processes.

Pinitol or D-chiro-inositol, which serves as an effective ingredient in the present disclosure, can be used for treating, preventing, or alleviating premenstrual syndrome.

"Premenstrual syndrome", which is the disease to be treated or alleviated in the present disclosure, refers to mental, behavioral, and physical symptoms that occur 7-10 days before menstruation and disappear or reduce with onset of a women's period. The symptoms mostly vanish or are alleviated when menstruation starts, but may not perish and may persist for some women during and even after their periods.

In this context, the term "pre- and postmenstrual syndrome" falls within the scope of the meaning of premenstrual syndrome as defined in the present disclosure.

Representative symptoms of premenstrual syndrome include physical symptoms such as headache, water stagnation in the body, etc., and mental symptoms such as depression, neurosis, food cravings, etc.

Concrete physical symptoms associated with premenstrual syndrome include tension-type headaches, tenderness in the breasts, bloating, swelling, abdominal cramps, generalized pain, weight gain, flushing, syncope, dizziness, nausea, fast heartbeat, and feeling tired, but are not limited thereto.

Mental symptoms of premenstrual syndrome include anxiety-related symptoms such as tension, mood swings, anger, dithering, etc., and anxious depression, loneliness, insomnia, animosity, wrath, amnesia, confusion, concentration impairment, distractibility, and the like, but are not limited to.

Behavioral symptoms associated with premenstrual syndrome may be exemplified by increased appetite, especially carvings for sweet or salty foods, avoidance of social or business activity, stay-at-home, and increase or decrease in interest in sex, but not limited to.

The composition of the present disclosure can treat, prevent, or alleviate one of the physical, mental, or behavioral symptoms of premenstrual syndrome.

The pharmaceutical composition of the present disclosure is described herein in the form of a composition for treating or preventing premenstrual syndrome, the composition comprising a pharmaceutically effective amount of (a) pinitol, D-chiro-inositol or an analog thereof; and (b) a pharmaceutically acceptable carrier.

As used herein, the term "prevention" or "preventing" is intended to mean the inhibition of onset of a disease or disorder in an animal which has not been diagnosed to possess the disease or disorder, but is liable to be affected thereby.

As used herein, the term "treatment" or "treating" is intended to mean (i) suppressing the development of a disease or disorder, (ii) alleviating symptoms of a disease or disorder, or (iii) eliminating a disease or disorder.

The pharmaceutically acceptable carrier contained in the pharmaceutical composition of the present disclosure is a typical one used in formulation, examples of which include carbohydrate compounds (e.g., lactose, amylose, dextrose, sucrose, sorbitol, mannitol, starch, cellulose, etc.), acacia gum, calcium phosphate, alginate, gelatin, calcium silicate, microcrystalline cellulose, polyvinylpyrrolidone, cellulose, water, syrup, salt solutions, alcohols, Arabic gum, vegetable oils (e.g., corn oil, cotton seed oil, soybean oil, olive oil, coconut oil), polyethylene glycol, methyl cellulose, methyl hydroxybenzoate, talc, magnesium stearate, and mineral oil, but are not limited thereto.

In addition to the ingredients, the pharmaceutical composition of the present disclosure may further comprise a lubricant, a wetting agent, a sweetener, a flavorant, an emulsifier, a suspending agent, a preservative, etc. With respect to details of suitable pharmaceutically acceptable carriers, reference may be made to Remington's Pharmaceutical Sciences (19th Ed., 1995).

A suitable dose of the pharmaceutical composition of the present disclosure may vary depending on various factors including the type of formulation, the modality of administration, the patient's age, weight, and sex, the severity of the disorder being treated, diet, the time of administration, the route of administration, the rate of excretion, and sensitivity. For example, the pharmaceutical composition of the present disclosure may range in oral dose from 0.001 to 1000 mg/kg of body weight.

According to methods that a person skilled in the art can easily carried out, the pharmaceutical composition of the present disclosure may be formulated with a pharmaceutically acceptable carrier and/or excipient into a unit dose form or before being included within a multiple dose package.

In this regard, the formulation may be in the form of a solution, suspension or emulsion in an oil or aqueous medium or in the form of an extract, a powder, a granule, a tablet, or a capsule, and may further comprise a dispersant or a stabilizer.

Also described herein is a method for preventing or treating premenstrual syndrome, the method comprising the step of administering to a subject a pharmaceutical composition comprising pinitol, D-chiro-inositol, or an analog thereof as an effective ingredient.

As used herein, the term "administration" or "administering' refer to directly administering a therapeutically effective amount of the composition of the present disclosure to a subject (individual) in need thereof to form the same amount in the body of the subject.

The term "therapeutically effective amount" of the composition refers to an enough amount of the composition to provide a therapeutic or prophylactic effect for a subject to be administered and is intended to encompass a "prophylactically effective amount". The term "subject", as used herein, is intended to encompass humans, mice, rats, guinea pigs, dogs, cats, horses, cow, pigs, monkeys, chimpanzees, baboons, and rhesus monkeys, with preference for humans.

For the method for prevention or treatment of premenstrual syndrome according to an aspect of the present disclosure, which is a method comprising administering the pharmaceutical composition for prevention or treatment premenstrual syndrome according to another aspect of the present disclosure, an overlapping contents therebetween are omitted in order to avoid exaggerated complexity in describing the specification.

Also described herein is a food composition comprising pinitol, D-chiro-inositol, or an analog thereof as an effective ingredient for preventing or alleviating premenstrual syndrome.

The food composition described herein may be in the form of a food composition for treating or preventing premenstrual syndrome, the composition comprising a sitologically effective amount of (a) pinitol, D-chiro-inositol, or an analog thereof; and (b) a sitologically acceptable carrier.

As used herein, the term "alleviating" or "alleviation" refer to reduce symptoms of a disease or symptoms due to a complication of the disease.

For use as a food functional composition, the food composition of the present disclosure may comprise an ingredient typically used therefor in addition to the effective ingredient. For example, a protein, a carbohydrate, a lipid, a nutrient, a flavoring agent, and a sweetener may be added.

Examples of the carbohydrate include typical saccharides inclusive of monosaccharides such as glucose, fructose, and the like; disaccharides such as maltose, sucrose, oligosaccharides and the like; and polysaccharides such as dextrin, cyclodextrin, and the like; and sugar alcohols such as xylitol, sorbitol, erythritol, and the like.

The sweetener may be a natural one [thaumatin, stevia extracts (e.g., rebaudioside A, glycyrrhizin, etc.)] or a synthetic one (saccharin, aspartame, etc.).

By way of example, when the food composition of the present disclosure is prepared to be a drink, it may further comprise citric acid, liquid fructose, sugar, glucose, acetic acid, malic acid, fruit juice, a eucommia extract, a jujube extract, a licorice extract, etc. in addition to the effective ingredient pinitol, D-chiro-inositol or an analog thereof.

In terms of the convenience of access to foods, the food composition of the present disclosure is very useful for preventing or alleviating premenstrual syndrome.

Features and advantages of the present disclosure are summarized as follows:
(i) The present disclosure relates to the use of pinitol, D-chiro-inositol or an analog thereof in preventing, alleviating, or treating premenstrual syndrome.
(ii) According to the present disclosure, pinitol, D-chiro-inositol or an analog thereof has the effect of alleviating dysmenorrhea, which is a main symptom of premenstrual syndrome, a period of dysmenorrhea, and coldness of hands and feet and thus can be developed into a drug or a nutraceutical for preventing, alleviating, or treating premenstrual syndrome.
(iii) D-Chiro-inositol, pinitol, or an analog thereof, which are all naturally occurring compounds, is less prone to elicit side effects when applied to the human body.

### Advantageous Effects

According to the present disclosure, pinitol, D-chiro-inositol or an analog thereof can be developed into a drug or a nutraceutical for prevention, alleviation, or treatment of premenstrual syndrome thanks to the alleviative effect thereof on the main symptoms of premenstrual syndrome inclusive of dysmenorrhea, a period of dysmenorrhea, and coldness of hands and feet, and may elicit few side effects when applied to the human body because they are derived from natural products.

### Brief Description of the Drawings

FIG. 1 shows results of alleviating effects on premenstrual syndrome after administration of a placebo and pinitol at a dose of 600 mg a day.
FIG. 2 shows results of alleviating effects on premenstrual syndrome after administration of a placebo and pinitol at a dose of 1,200 mg a day.
FIG. 3 shows results of alleviating effects on premenstrual syndrome after administration of a placebo and pinitol at a dose of 2,400 mg a day.
FIG. 4 shows results of alleviating effects on premenstrual syndrome after administration of a placebo and D-chiro-inositol (DCI) at a dose of 600 mg a day.
FIG. 5 shows results of alleviating effects on premenstrual syndrome after administration of a placebo and D-chiro-inositol (DCI) at a dose of 1,200 mg a day.
FIG. 6 shows results of alleviating effects on premenstrual syndrome after administration of a placebo and D-chiro-inositol (DCI) at a dose of 2,400 mg a day.

### Mode for Carrying Out the Invention

A better understanding of the present invention may be obtained through the following examples which are set forth to illustrate, but are not to be construed as limiting the present invention.

### EXAMPLES

### Experiment Methods

### 1. Evaluation of Administration of Pinitol or D-Chiro-Inositol to Female Subject

Pinitol and D-chiro-inositol were purchased from Biosearch Life (Camino de Purchil, 66. 18004 Granada, Spain). Selected as experiment subjects were 80 normal women of reproductive age, that is, 80 women at the age of 25-40 who had neither skipped three or more cycles of menstruation, nor been administered with a contraceptive pill, and nor received a psychiatric treatment.

Of them, 40 persons were classified into four groups of 10 which were then administered respectively with a placebo free of pinitol and having no pharmaceutical effects and with pinitol at three different doses. Likewise, the remaining 40 persons were equally divided into one placebo group and three experimental groups to which D-chiro-inositol was administered at three different doses, respectively. A blind test was conducted in which the placebo and pinitol or D-chiro-inositol was administered once a day for eight weeks. Pinitol or D-chiro-inositol was administered at daily doses of 600 mg, 1,200 mg, and 2,400 mg for eight weeks. A survey was made of the experimental subjects before and after the clinical test of placebo and pinitol or D-chiro-inositol to evaluate the alleviating effect of pinitol or D-chiro-inositol on premenstrual syndrome.

### 2. Survey

In the present disclosure, the questionnaire that experimental subjects had completed with respect to the severity of premenstrual syndrome were analyzed. The experimental subjects evaluated the placebo, pinitol, or D-chiro-inositol for a pharmacological effect on premenstrual syndrome on the basis of four grades: complete alleviation, significant alleviation, somewhat alleviation, and no change for dysmenorrhea, a period of dysmenorrhea, and coldness of hands and feet. The questionnaire used in the present disclosure is given in Table 1.

**[Tables 1]**

| symptom | condition grades | | | |
|---|---|---|---|---|
| | no change | somewhat alleviation | significant alleviation | complete alleviation |
| 1. dysmenorrhea | | | | |
| 2. period of dysmenorrhea | | | | |
| 3. coldness of hands and feet | | | | |

### Experimental Results

### 1. Alleviative Effect of Pinitol or D-Chiro-Inositol on Premenstrual Syndrome

A survey was made of women to evaluate the alleviative effect of pinitol or D-chiro-inositol on premenstrual syndrome. For the control administered with the placebo, main symptoms of premenstrual syndrome, such as dysmenorrhea, a period of dysmenorrhea, and coldness of hands and feet, were alleviated by 10-20 %. Significant reductions in the main symptoms of premenstrual syndrome, such as dysmenorrhea, a period of dysmenorrhea, and coldness of hands and feet were detected in the experimental groups administered with pinitol or D-chiro-inositol at a daily dose of 600 mg, 1,200 mg, and 2,400 mg (Tables 2 and 3). Questionnaire results are shown in Table 2 after the administration of placebo or pinitol and in Table 3 after the administration of placebo or D-chiro-inositol (DCI).

**[Tables 2]**

| classification | | complete alleviation | | significant alleviation | | alleviation | | no change | |
|---|---|---|---|---|---|---|---|---|---|
| | | the number of people (person) | percentage (%) | the number of people (person) | percentage (%) | the number of people (person) | percentage (%) | the number of people (person) | percentage (%) |
| placebo | dysmenorrhea | 0 | 0.0 | 0 | 0.0 | 1 | 10.0 | 9 | 90.0 |
| | period of dysmenorrhea | 0 | 0.0 | 0 | 0.0 | 2 | 20.0 | 8 | 80.0 |
| | coldness of hands and feet | 0 | 0.0 | 0 | 0.0 | 1 | 10.0 | 9 | 90.0 |
| Pinitol (600mg) | dysmenorrhea | 0 | 0.0 | 3 | 30.0 | 3 | 30.0 | 4 | 40.0 |
| | period of dysmenorrhea | 0 | 0.0 | 3 | 30.0 | 2 | 20.0 | 5 | 50.0 |
| | coldness of hands and feet | 0 | 0.0 | 1 | 10.0 | 4 | 40.0 | 5 | 50.0 |
| Pinitol (1,20mg) | dysmenorrhea | 6 | 60.0 | 2 | 20.0 | 1 | 10.0 | 1 | 10.0 |
| | period of dysmenorrhea | 5 | 50.0 | 2 | 20.0 | 2 | 20.0 | 1 | 10.0 |
| | coldness of hands and feet | 4 | 40.0 | 3 | 30.0 | 1 | 10.0 | 2 | 20.0 |
| Pinitol (2,400mg) | dysmenorrhea | 8 | 80.0 | 2 | 20.0 | 0 | 0.0 | 0 | 0.0 |
| | period of dysmenorrhea | 8 | 80.0 | 2 | 20.0 | 0 | 0.0 | 0 | 0.0 |
| | coldness of hands and feet | 7 | 70.0 | 1 | 10.0 | 1 | 10.0 | 1 | 10.0 |

**[Tables 3]**

| classification | | complete alleviation | | significant alleviation | | alleviation | | no change | |
|---|---|---|---|---|---|---|---|---|---|
| | | the number of people (person) | percentage (%) | the number of people (person) | percentage (%) | the number of people (person) | percentage (%) | the number of people (person) | percentage (%) |
| placebo | dysmenorrhea | 0 | 0.0 | 0 | 0.0 | 2 | 20.0 | 8 | 80.0 |
| | period of dysmenorrhea | 0 | 0.0 | 0 | 0.0 | 2 | 20.0 | 8 | 80.0 |
| | coldness of hands and feet | 0 | 0.0 | 0 | 0.0 | 1 | 10.0 | 9 | 90.0 |
| DCI (600mg) | dysmenorrhea | 0 | 0.0 | 4 | 40.0 | 2 | 20.0 | 4 | 40.0 |
| | period of dysmenorrhea | 0 | 0.0 | 3 | 30.0 | 2 | 20.0 | 5 | 50.0 |
| | coldness of hands and feet | 0 | 0.0 | 2 | 20.0 | 3 | 30.0 | 5 | 50.0 |
| DCI (1,20 0mg) | dysmenorrhea | 5 | 50.0 | 3 | 30.0 | 2 | 20.0 | 0 | 0.0 |
| | period of dysmenorrhea | 6 | 60.0 | 2 | 20.0 | 1 | 10.0 | 1 | 10.0 |
| | coldness of hands and feet | 5 | 50.0 | 2 | 20.0 | 2 | 20.0 | 1 | 10.0 |
| DCI (2,40 0mg) | dysmenorrhea | 9 | 90.0 | 1 | 10.0 | 0 | 0.0 | 0 | 0.0 |
| | period of dysmen orrhea | 8 | 80.0 | 2 | 20.0 | 0 | 0.0 | 0 | 0.0 |
| | coldness of hands and feet | 8 | 80.0 | 1 | 10.0 | 0 | 0.0 | 1 | 10.0 |

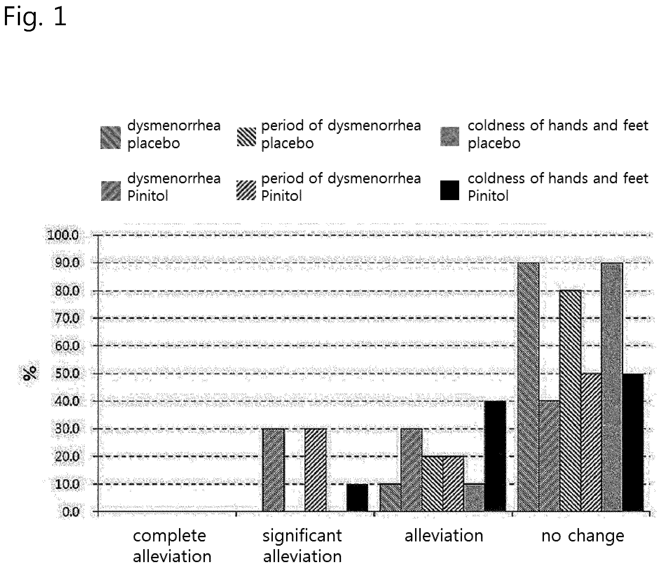

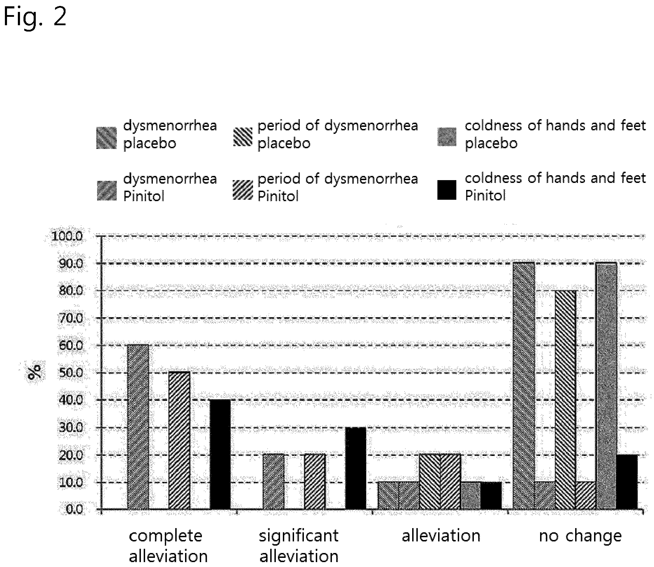

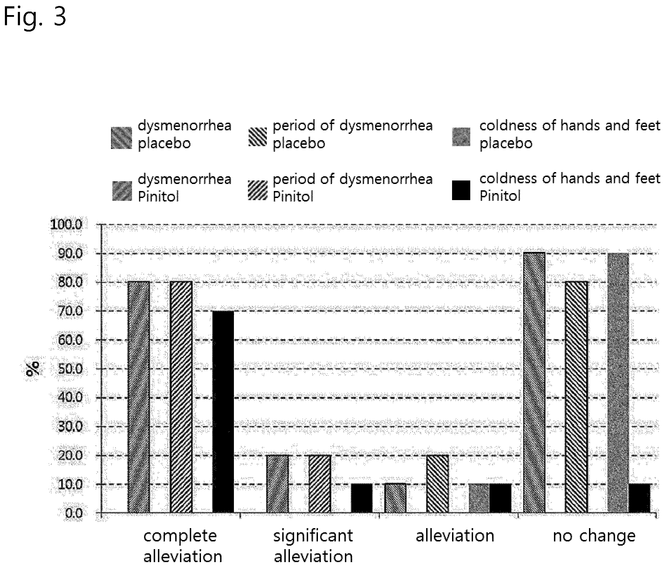

As many as 50-60 % of the administration group of 600 mg pinitol experienced somewhat or significant alleviation of the main symptoms of premenstrual syndrome before administration. Only 10 % of the administration group of pinitol 1,200 mg or 2,400 mg did not feel changes in the main symptoms of premenstrual syndrome whereas alleviation was answered by as many as 80-90% of the group, with the complete alleviation found in 50 % or more of the administration group of 1,200 mg and in 70-80 % in the administration group of 2,400 mg (see FIGS. 1-3).

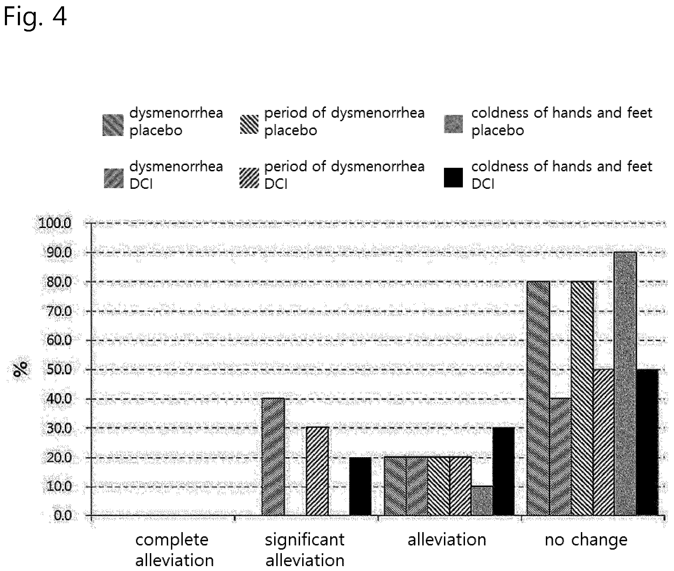

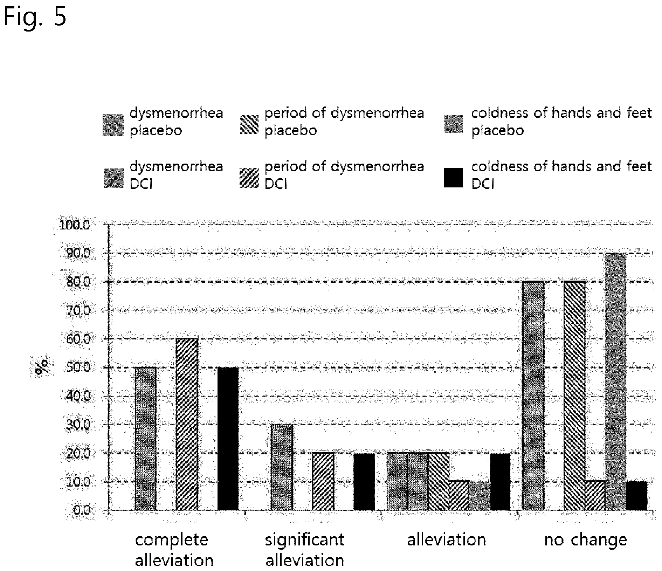

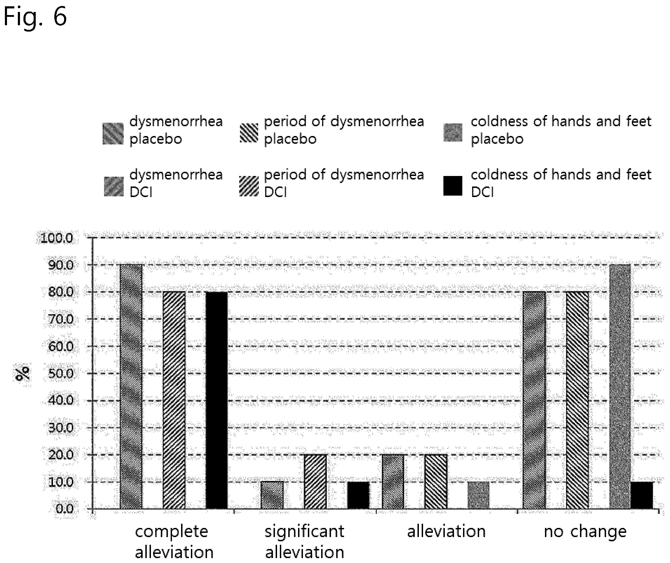

The main symptoms of premenstrual syndrome were somewhat or significantly alleviated in 50-60 % of the administration group of D-chiro-inositol 600 mg, compared to pre-administration results, like the result of the administration group of pinitol 600 mg. Similar results to those of the administration groups of pinitol 1,200 mg or 2,400 mg were obtained in the administration groups of D-chiro-pinitol. That is, only 10 % of the administration group of D-chiro-pinitol 1,200 mg or 2,400 mg did not experience changes in the main symptoms of premenstrual syndrome whereas 80-90% of the group answered alleviation in the main symptoms, with the complete alleviation found in 50 % or more of the administration group of 1,200 mg and in 70-80 % in the administration group of 2,400 mg (see FIGS. 4-6).

The analysis result of the survey indicates that pinitol and D-chiro-inositol has the effect of significantly alleviating the main symptoms of premenstrual syndrome such as dysmenorrhea, a period of dysmenorrhea, and coldness of hands and feet.

## Claims

1. A pharmaceutical composition comprising pinitol or D-chiro-inositol as an effective ingredient for use in the prevention or treatment of premenstrual syndrome.

2. The pharmaceutical composition for use according to claim 1, wherein pinitol or D-chiro-inositol is contained in a concentration of 50 - 5000 mg in the entire composition.

3. The pharmaceutical composition for use according to claim 1, wherein the premenstrual syndrome includes a physical symptom or a mental symptom.

4. The pharmaceutical composition for use according to claim 3, wherein the physical symptom of premenstrual syndrome includes at least one among tension-type headaches, tenderness in the breasts, bloating, swelling, abdominal cramps, generalized pain, weight gain, flushing, syncope, dizziness, nausea, fast heartbeat, and feeling tired.

5. The pharmaceutical composition for use according to claim 3, wherein the mental symptom of premenstrual syndrome includes at least one among anxiety-related symptoms inclusive of tension, mood swings, anger, and dithering; anxious depression, loneliness, insomnia, animosity, wrath, amnesia, confusion, concentration impairment, and distractibility.

6. A food composition comprising pinitol or D-chiro-inositol as an effective ingredient for use in alleviating premenstrual syndrome.

7. The food composition for use according to claim 6, wherein pinitol or D-chiro-inositol is contained in a concentration of 50 - 5000 mg in the entire composition.

8. The food composition for use according to claim 6, wherein the premenstrual syndrome includes a physical symptom or a mental symptom.

9. The food composition for use according to claim 8, wherein the physical symptom of premenstrual syndrome includes at least one among tension-type headaches, tenderness in the breasts, bloating, swelling, abdominal cramps, generalized pain, weight gain, flushing, syncope, dizziness, nausea, fast heartbeat, and feeling tired.

10. The food composition for use according to claim 8, wherein the mental symptom of premenstrual syndrome includes at least one among anxiety-related symptoms inclusive of tension, mood swings, anger, and dithering; anxious depression, loneliness, insomnia, animosity, wrath, amnesia, confusion, concentration impairment, and distractibility.

## Patentansprüche

1. Pharmazeutische Zusammensetzung, umfassend Pinitol oder D-Chiro-Inositol als wirksamen Inhaltsstoff zur Verwendung bei der Prävention oder Behandlung von prämenstruellem Syndrom.

2. Pharmazeutische Zusammensetzung zur Verwendung nach Anspruch 1, wobei Pinitol oder D-Chiro-Inositol in einer Konzentration von 50-5000 mg in der gesamten Zusammensetzung enthalten ist.

3. Pharmazeutische Zusammensetzung zur Verwendung nach Anspruch 1, wobei das prämenstruelle Syndrom ein physisches Symptom oder ein mentales Symptom beinhaltet.

4. Pharmazeutische Zusammensetzung zur Verwendung nach Anspruch 3, wobei das physische Symptom von prämenstruellem Syndrom zumindest eines von Spannungskopfschmerzen, empfindlichen Brüsten, Völlegefühl, Schwellung, Bauchkrämpfen, allgemeinen Schmerzen, Gewichtszunahme, Hitzegefühl, Ohnmacht, Schwindel, Übelkeit, schnellem Herzschlag und Müdigkeitsgefühl beinhaltet.

5. Pharmazeutische Zusammensetzung zur Verwendung nach Anspruch 3, wobei das mentale Symptom von prämenstruellem Syndrom zumindest eines von mit Angst verbundenen Symptomen einschließlich Anspannung, Stimmunsschwankungen, Wut und Unruhe; ängstlicher Depression, Einsamkeit, Schlaflosigkeit, Feindseligkeit, Zorn, Amnesie, Verwirrtheit, Konzentrationsbeeinträchtigung und Ablenkbarkeit beinhaltet.

6. Nahrungsmittelzusammensetzung, umfassend Pinitol oder D-Chiro-Inositol als wirksamen Inhaltsstoff zur Verwendung bei der Linderung von prämenstruellem Syndrom.

7. Nahrungsmittelzusammensetzung zur Verwendung nach Anspruch 6, wobei Pinitol oder D-Chiro-Inositol in einer Konzentration von 50-5000 mg in der gesamten Zusammensetzung enthalten ist.

8. Nahrungsmittelzusammensetzung zur Verwendung nach Anspruch 6, wobei das prämenstruelle Syndrom ein physisches Symptom oder ein mentales Symptom beinhaltet.

9. Nahrungsmittelzusammensetzung zur Verwendung nach Anspruch 8, wobei das physische Symptom von prämenstruellem Syndrom zumindest eines von Spannungskopfschmerzen, empfindlichen Brüsten, Völlegefühl, Schwellung, Bauchkrämpfen, allgemeinen Schmerzen, Gewichtszunahme, Hitzegefühl, Ohnmacht, Schwindel, Übelkeit, schnellem Herzschlag und Müdigkeitsgefühl beinhaltet.

10. Nahrungsmittelzusammensetzung zur Verwendung nach Anspruch 8, wobei das mentale Symptom von prämenstruellem Syndrom zumindest eines von mit Angst verbundenen Symptomen einschließlich Anspannung, Stimmunsschwankungen, Wut und Unruhe; ängstlicher Depression, Einsamkeit, Schlaflosigkeit, Feindseligkeit, Zorn, Amnesie, Verwirrtheit, Konzentrationsbeeinträchtigung und Ablenkbarkeit beinhaltet.

## Revendications

1. Composition pharmaceutique comprenant du pinitol ou du D-chiro-inositol comme un ingrédient efficace, destinée à être utilisée dans la prévention ou le traitement du syndrome prémenstruel.

2. Composition pharmaceutique destinée à être utilisée selon la revendication 1, dans laquelle le pinitol ou le D-chiro-inositol est contenu dans une concentration de 50 à 5 000 mg dans la composition entière.

3. Composition pharmaceutique destinée à être utilisée selon la revendication 1, dans laquelle le syndrome prémenstruel inclut un symptôme physique ou un symptôme mental.

4. Composition pharmaceutique destinée à être utilisée selon la revendication 3, dans laquelle le symptôme physique du syndrome prémenstruel inclut au moins l'un parmi des céphalées de type tension, la sensibilité des seins, le ballonnement, le gonflement, les crampes abdominales, la douleur généralisée, la prise de poids, les bouffées de chaleur, la syncope, le vertige, la nausée, le pouls rapide, et la sensation de fatigue.

5. Composition pharmaceutique destinée à être utilisée selon la revendication 3, dans laquelle le symptôme mental du syndrome prémenstruel inclut au moins l'un parmi les symptômes relatifs à l'anxiété incluant la tension, les sautes d'humeur, la colère, et la l'indécision ; la dépression anxieuse, la solitude, l'insomnie, l'animosité, la fureur, l'amnésie, la confusion, l'altération de la concentration, et la distractivité.

6. Composition alimentaire comprenant du pinitol ou du D-chiro-inositol comme un ingrédient effectif, destinée à être utilisée dans l'atténuation du syndrome prémenstruel.

7. Composition alimentaire destinée à être utilisée selon la revendication 6, dans laquelle le pinitol ou le D-chiro-inositol est contenu dans une concentration de 50 à 5 000 mg dans la composition entière.

8. Composition alimentaire destinée à être utilisée selon la revendication 6, dans laquelle le syndrome prémenstruel inclut un symptôme physique ou un symptôme mental.

9. Composition alimentaire destinée à être utilisée selon la revendication 8, dans laquelle le symptôme physique du syndrome prémenstruel inclut au moins l'un parmi des céphalées de type tension, la sensibilité des seins, le ballonnement, le gonflement, les crampes abdominales, la douleur généralisée, la prise de poids, les bouffées de chaleur, la syncope, le vertige, la nausée, le pouls rapide, et la sensation de fatigue.

10. Composition alimentaire destinée à être utilisée selon la revendication 8, dans laquelle le symptôme mental du syndrome prémenstruel inclut au moins l'un parmi les symptômes relatifs à l'anxiété incluant la tension, les sautes d'humeur, la colère, et la l'indécision ; la dépression anxieuse, la solitude, l'insomnie, l'animosité, la fureur, l'amnésie, la confusion, l'altération de la concentration, et la distractivité.
